# EUROPEAN PATENT SPECIFICATION

(11) **EP 3 552 600 B1**
(45) Date of publication and mention of the grant of the patent: **17.02.2021**
(21) Application number: 19168805.0
(22) Date of filing: 11.04.2019
(51) Int. Cl.: A61K 8/73, A61K 8/9783, A61Q 19/00, A61Q 19/08

(54) **COMPOSITION CONTAINING DENDROBIUM CANDIDUM EXTRACT AND PREPARATION METHOD AND USE THEREOF**
ZUSAMMENSETZUNG MIT DENDROBIUM-CANDIDUM-EXTRAKT SOWIE HERSTELLUNGSVERFAHREN UND VERWENDUNG DAVON
COMPOSITION CONTENANT UN EXTRAIT DE DENDROBIUM CANDIDUM, SON PROCÉDÉ DE PRÉPARATION ET SON UTILISATION

(30) Priority: 12.04.2018 CN 201810325789
(43) Date of publication of application: 16.10.2019
(73) Proprietor: Shanghai Traditional Chinese Medicine Co., Ltd., Shanghai 200002 (CN)
(72) Inventor: ZHANG, Xue, Huangpu District Shanghai 200002 (CN); LIU, Guodong, Huangpu District Shanghai 200002 (CN); SI, Ruirong, Huangpu District Shanghai 200002 (CN)
(74) Representative: Hanna Moore + Curley

(56) References cited:
- CN-A- 106 511 220
- CN-A- 106 727 148
- CN-A- 107 811 939
- CN-B- 104 127 341
- KUMUD MADAN ET AL: "In-vitro evaluation of antioxidant, anti-elastase, anti-collagenase, anti-hyaluronidase activities of safranal and determination of its sun protection factor in skin photoaging", BIOORGANIC CHEMISTRY., vol. 77, 1 April 2018 (2018-04-01), pages 159-167, XP055615449, US ISSN: 0045-2068, DOI: 10.1016/j.bioorg.2017.12.030

## Description

### FIELD OF THE INVENTION

The present invention relates to a composition containing *Dendrobium candidum* extract and a preparation method and use thereof.

### BACKGROUND OF THE INVENTION

*Dendrobium candidum,* as a plant belonging to *Dendrobium* genus in the *Orchidaceae* family, is commonly used in the treatment of diseases such as deficiencies of Yin and body fluid, dry mouth and polydipsia, anorexia and vomiturition, asthenia fever after illness, and blurred vision, etc. Modern scientific research has proved that the main active components contained in *Dendrobium candidum* are *Dendrobium* polysaccharides, dendrobine, amino acids, stilbenes and their derivatives as well as a variety of volatile components.

Modern research has proven that *Dendrobium candidum* has effects of anti-aging, anti-tumor, reducing blood glucose and improving immune function, etc., and has a good effect on diseases such as malignant tumors, gastrointestinal diseases, diabetes, cataracts, arthritis, thromboangiitis obliterans and chronic pharyngitis, therefore, it is called "the giant panda in the pharmaceutical industry" by the international medicinal plant community.

Studies have found that *Dendrobium candidum* can significantly increase the activities of superoxide dismutase, catalase, oxide enzyme and ascorbate peroxidase, and has different antioxidant abilities in different tissues and organs at different times (LI Biao, WEI Jinmin, WEI Xiaolan. Effect of sound wave stress on antioxidant enzyme activities and lipid peroxidation of Dendrobium candidum[J]. Colloids and Surfaces B: Biointerfaces, 2008, 63: 269-275.). It has also been found that *Dendrobium candidum* extract could be used in the treatment of patients with dry mouth symptoms, wherein the salivary secretion in patients in the treatment group increased by about 65%, and high expression of water channel protein aquaporin-5 (AQP-5) was found in the biopsies of patients in the treatment group, indicating that *Dendrobium candidum* could regulate the expression of AQP-5 and promote salivation in patients with dry mouth symptoms (LIN Xiao, TZI B N, YI Binfeng. Dendrobium candidum extract increases the expression of aquaporin-5 in labia glands from patients with Sjögren's syndrome[J]. Phytomedicine, 2011, 18: 194-198.).

*Dendrobium candidum* has excellent efficacy in nourishing Yin and generating body fluid, and moistening dryness. In modern research, the material basis of its efficacy has been further clarified, which greatly improves its safety. At present, *Dendrobium candidum* is a very popular raw material for cosmetics. However, when the *Dendrobium candidum* extract is used alone, it is difficult to maintain its moisturizing effect for a long time. Therefore, the development of a facial mask containing *Dendrobium candidum* extract which can maintain the moisturizing effect for a long time is of great commercial value, which is also an urgent problem to be solved in the art.

The state of the art is represented by Chinese patent specifications CN106511220A, CN107811939A, CN106727148A and CN104127341B, and scientific publication Kumud Madan et al., "In-vitro evaluation of antioxidant, anti-elastase, anti-collagenase, anti-hyaluronidase activities of safranal and determination of its sun protection factor in skin photoaging" Bioorganic Chemistry, vol. 77, 2018, pages 159-167.

### SUMMARY OF THE INVENTION

In order to overcome the defects in the prior art that the *Dendrobium candidum* extract, when used alone, does not have a long-lasting moisturizing effect, the present invention provides a composition containing *Dendrobium candidum* extract and a preparation method and use thereof. The facial mask prepared with the composition containing *Dendrobium candidum* extract provided by the present invention has a good hydrating effect and long-lasting moisturizing effect.

The present invention solves the above technical problems by the following technical solutions.

The present invention provides a composition containing *Dendrobium candidum* extract, which comprises the following raw materials in parts by mass: 4-6 parts of sodium hyaluronate, 0.5-1.5 parts of *Dendrobium candidum* stem extract, 0.5-1.5 parts of *Crocus sativus* extract, 0.5-1.5 parts of *Cereus grandiflorus* extract, 0.5-1.5 parts of *Centaurea cyanus* extract and 0.5-1.5 parts of *Scutellaria baicalensis* root extract.

In the present invention, the sodium hyaluronate may be a conventional sodium hyaluronate in the art, preferably purchased from Bloomage Freda Biopharm Co., Ltd.

In the present invention, the *Dendrobium candidum* stem extract may be obtained by a conventional method in the art, and is generally obtained by the following method: mixing the stem of *Dendrobium candidum* with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Dendrobium candidum* is produced from a planting base for *Dendrobium candidum* of Shanghai traditional Chinese medicine Co., Ltd. in Yunnan.

In the present invention, the *Crocus sativus* extract may be obtained by a conventional method in the art, and is generally obtained by the following method: mixing *Crocus sativus* L with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Crocus sativus* L is produced from a professional planting base for *Crocus sativus* L of Shanghai traditional Chinese medicine Co., Ltd. in Chongming Island.

In the present invention, the *Cereus grandiflorus* extract may be obtained by a conventional method in the art, and is generally obtained by the following method: mixing *Cereus grandiflorus* (cactus) with a solvent and then extracting it by a conventional method in the art. The solvent may be a conventional extraction solvent in the art, preferably one or more of water, ethanol and 1,3-butanediol. The extraction method may be a dipping method, a percolating method, a decocting method, a refluxing method or a continuous extraction method, etc. Preferably, the *Cereus grandiflorus* extract is purchased from Liaoyuan Daily Chemical Co., Ltd.

In the present invention, the *Centaurea cyanus* extract may be obtained by a conventional method in the art, and is generally obtained by the following method: mixing *Centaurea cyanus* with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Centaurea cyanus* extract is purchased from Liaoyuan Daily Chemical Co., Ltd.

In the present invention, the *Scutellaria baicalensis* root extract may be obtained by a conventional method in the art, and is generally obtained by the following method: mixing the root of *Scutellaria baicalensis* with a solvent and then extracting it by a conventional method in the art. The solvent may be a conventional extraction solvent in the art, preferably water and/or ethanol. The extraction method may be a dipping method, a percolating method, a decocting method, a refluxing method or a continuous extraction method, etc. Preferably, the *Scutellaria baicalensis* root extract is purchased from Liaoyuan Daily Chemical Co., Ltd.

In the present invention, the content of the sodium hyaluronate is preferably 5 parts.

In the present invention, the content of the *Dendrobium candidum* stem extract is preferably 1 part.

In the present invention, the content of the *Crocus sativus* extract is preferably 1 part.

In the present invention, the content of the *Cereus grandiflorus* extract is preferably 1 part.

In the present invention, the content of the *Centaurea cyanus* extract is preferably 1 part.

In the present invention, the content of the *Scutellaria baicalensis* root extract is preferably 1 part.

In the present invention, the composition containing *Dendrobium candidum* extract may further comprise one or more of a solvent, a humectant, a thickener, a pH adjuster, a solubilizer, an antioxidant, a skin conditioner, a preservative, and a fragrance.

Wherein, the solvent may be a conventional one in the art, such as water.

Wherein, the humectant may be a conventional one in the art, such as propylene glycol.

Wherein, the content of the humectant may be a conventional content in the art, for example, the mass ratio of the humectant to the *Dendrobium candidum* stem extract is preferably (1400 to 1600):1, and more preferably 1500:1.

Wherein, the thickener may be a conventional one in the art, such as hydroxyethyl cellulose.

Wherein, the content of the thickener may be a conventional content in the art, for example, the mass ratio of the thickener to the *Dendrobium candidum* stem extract is preferably (30 to 50):1, and more preferably 40:1.

Wherein, the pH adjuster may be a conventional one in the art, such as triethanolamine.

Wherein, the content of the pH adjuster may be a conventional content in the art, which is a content of the pH adjuster required to adjust to a suitable pH range according to the common knowledge in the art.

Wherein, the solubilizer may be a conventional one in the art, such as PEG-40 hydrogenated castor oil.

Wherein, the content of the solubilizer may be a conventional content in the art, for example, the mass ratio of the solubilizer to the *Dendrobium candidum* stem extract is preferably (4 to 6):1, and more preferably 5:1.

Wherein, the antioxidant may be a conventional one in the art, such as tocopheryl acetate.

Wherein, the content of the antioxidant may be a conventional content in the art, for example, the mass ratio of the antioxidant to the *Dendrobium candidum* stem extract is preferably (0.5 to 1.5):1, and more preferably 1:1.

Wherein, the skin conditioner may be a conventional one in the art, such as dipotassium glycyrrhizinate and/or hydrolyzed silk.

The hydrolyzed silk may be obtained by a conventional method in the art, and is generally obtained by hydrolyzing a cocoon shell to silk fibroin. Preferably, the hydrolyzed silk is purchased from Liaoyuan Daily Chemical Co., Ltd.

Wherein, the content of the skin conditioner is a conventional content in the art, and the mass ratio of the skin conditioner to the *Dendrobium candidum* stem extract is preferably (1 to 11):1, for example, 1:1, 10:1, and 11:1.

Wherein, the preservative can be a conventional one in the art, such as chlorphenesin.

Wherein, the content of the preservative is in compliance with relevant laws and regulations, and is a conventional content in the art, for example, the mass ratio of the preservative to the *Dendrobium candidum* stem extract is preferably (15 to 25):1, and more preferably 20:1.

Wherein, the content of the fragrance may be a conventional content in the art, for example, the mass ratio of the fragrance to the *Dendrobium candidum* stem extract is preferably (0.5 to 1.5):1, and more preferably 1:1.

In a preferred embodiment of the present invention, the composition containing *Dendrobium candidum* extract comprises the following raw materials in parts by weight: 5 parts of sodium hyaluronate, 1 part of *Dendrobium candidum* stem extract, 1 part of *Crocus sativus* extract, 1 part of *Cereus grandiflorus* extract, 1 part of *Centaurea cyanus* extract and 1 part of *Scutellaria baicalensis* root extract.

In a preferred embodiment of the present invention, the composition containing *Dendrobium candidum* extract comprises: 84.11% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Dendrobium candidum* stem extract, 0.01% *Crocus sativus* extract, 0.01% *Cereus grandiflorus* extract, 0.01% *Centaurea cyanus* extract, 0.01% *Scutellaria baicalensis* root extract, 0.01% hydrolyzed silk, 0.1% dipotassium glycyrrhizinate, 0.2% chlorphenesin and 0.01% fragrance, and the above percentages refer to the mass percentage accounting for the total amount of the raw materials.

The present invention also provides a preparation method of the above composition containing *Dendrobium candidum* extract, in which the raw material components of the composition containing *Dendrobium candidum* extract are mixed.

When the raw materials contain other component(s) in addition to the sodium hyaluronate, the *Dendrobium candidum* stem extract, the *Crocus sativus* extract, the *Cereus grandiflorus* extract, the *Centaurea cyanus* extract and the *Scutellaria baicalensis* root extract, the other component(s) is/are firstly mixed, and then mixed with "the sodium hyaluronate, the *Dendrobium candidum* stem extract, the *Crocus sativus* extract, the *Cereus grandiflorus* extract, the *Centaurea cyanus* extract and the *Scutellaria baicalensis* root extract".

The present invention also provides use of the above composition containing *Dendrobium candidum* extract in cosmetics, such as a facial mask.

Wherein, the raw materials of the facial mask are preferably: a solvent, a humectant, a thickener, a pH adjuster, a solubilizer, an antioxidant, sodium hyaluronate, *Dendrobium candidum* stem extract, *Crocus sativus* extract, *Cereus grandiflorus* extract, *Centaurea cyanus* extract, *Scutellaria baicalensis* root extract, a skin conditioner, a preservative, and a fragrance.

The definitions of the solvent, the humectant, the thickener, the pH adjuster, the solubilizer, the antioxidant, the sodium hyaluronate, the *Dendrobium candidum* stem extract, the *Crocus sativus* extract, the *Cereus grandiflorus* extract, the *Centaurea cyanus* extract, the *Scutellaria baicalensis* root extract, the skin conditioner, the preservative, and the fragrance are as described above.

The raw materials of the facial mask are preferably the following components: 84.11% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Dendrobium candidum* stem extract, 0.01% *Crocus sativus* extract, 0.01% *Cereus grandiflorus* extract, 0.01% *Centaurea cyanus* extract, 0.01% *Scutellaria baicalensis* root extract, 0.01% hydrolyzed silk, 0.1% dipotassium glycyrrhizinate, 0.2% chlorphenesin and 0.01% fragrance, and the above percentages refer to the mass percentage accounting for the total amount of the raw materials.

The preparation method of the facial mask includes the following steps:
(1) mixing a group A and a group B, and homogenizing to obtain a mixture 1; the group A includes the humectant, and the group B includes the thickener, the pH adjuster, the sodium hyaluronate, the skin conditioner and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance; and
(4) mixing the mixture 3 in the step (3) with a group D, and homogenizing; and the group D includes the *Dendrobium candidum* stem extract, the *Crocus sativus* extract, the *Cereus grandiflorus* extract, the *Centaurea cyanus* extract and the *Scutellaria baicalensis* root extract.

In the step (1), the method for mixing the group A and the group B may be a conventional one in the art, for example, mixing the raw materials; preferably, separately adding the raw materials of the group B to the group A; and more preferably, separately adding the raw materials of the group B to the group A after homogenization. The time for homogenizing may be a conventional one in the art, for example, 20 min.

In the step (1), the time for homogenizing is preferably 25 min.

In the step (1), preferably, the mixture 1 is filtered. The mesh number of the filter screen for filtration may be a conventional one in the art, for example, 300 mesh.

In the step (2), the time for homogenizing is preferably 60 to 90 min.

In the step (3), the preparation method of the group C may be a conventional one in the art, for example, uniformly stirring the solubilizer, the antioxidant and the fragrance. The time for stirring may be a conventional one in the art, for example, 10 min.

In the step (3), the time for homogenizing is preferably 25 min.

In the step (4), preferably, the *Dendrobium candidum* stem extract, the *Crocus sativus* extract, the *Cereus grandiflorus* extract, the *Centaurea cyanus* extract and the *Scutellaria baicalensis* root extract are separately added to the mixture 3.

In the step (4), the time for homogenizing is preferably 20 min.

Based on the common knowledge in the art, the above various preferred conditions may be arbitrarily combined to obtain preferred embodiments of the present invention.

The reagents and raw materials used in the present invention are commercially available.

The beneficial effects of the present invention are that:
(1) The composition containing *Dendrobium candidum* extract prepared by the present application has long-lasting anti-oxidation and moisturizing effect. The prepared facial mask has a good hydrating and moisturizing effect, and after applying the facial mask continuously for four weeks and then suspending use for three weeks, the skin may remain the equivalent moisture content as it did after one week of use.
(2) The preparation process adopted in the present application is simple. Identification and risk assessment were carried out on the safety risk substances of the facial mask, and no safety risk was found in the use of the product.

### DETAILED DESCRIPTION OF THE INVENTION

The invention is further illustrated by the following examples, which are not intended to limit the invention. The experimental methods in the following examples, which do not specify the specific conditions, are selected according to conventional methods and conditions, or according to the manufacturer's instructions.

In the following examples, sodium hyaluronate was purchased from Bloomage Freda Biopharm Co., Ltd.

In the following examples, *Cereus grandiflorus* extract, *Centaurea cyanus* extract and *Scutellaria baicalensis* root extract were all purchased from Liaoyuan Daily Chemical Co., Ltd.

In the following examples, the preparation method of the *Crocus sativus* extract was: using *Crocus sativus* L produced from the professional planting base for *Crocus sativus* L of Shanghai traditional Chinese medicine Co., Ltd. in Chongming Island, and extracting it according to a conventional method in the art. The extraction solvent may be water and/or ethanol. The extraction method may be a dipping method, a percolating method, a decocting method, a refluxing method or a continuous extraction method, etc.

In the following examples, the preparation method of the *Dendrobium candidum* extract was: using *Dendrobium candidum* produced from the planting base for *Dendrobium candidum* of Shanghai traditional Chinese medicine Co., Ltd. in Yunnan, and extracting it according to a conventional method in the art. The extraction solvent may be water and/or ethanol. The extraction method may be a dipping method, a percolating method, a decocting method, a refluxing method or a continuous extraction method, etc.

### Example 1

In this example, the formula of the composition containing *Dendrobium candidum* extract and the parts by mass of each component were: sodium hyaluronate, 5 parts; *Dendrobium candidum* stem extract, 1 part; *Crocus sativus* extract, 1 part; *Cereus grandiflorus* extract, 1 part; *Centaurea cyanus* extract, 1 part; and *Scutellaria baicalensis* root extract, 1 part.

The composition was prepared by mixing sodium hyaluronate, *Dendrobium candidum* stem extract, *Crocus sativus* extract, *Cereus grandiflorus* extract, *Centaurea cyanus* extract and *Scutellaria baicalensis* root extract according to their mass ratios.

### Example 2

In this example, the formula of the composition containing *Dendrobium candidum* extract and the parts by mass of each component were: sodium hyaluronate, 5 parts; *Dendrobium candidum* stem extract, 1 part; *Crocus sativus* extract, 1 part; *Cereus grandiflorus* extract, 1 part; *Centaurea cyanus* extract, 1 part; *Scutellaria baicalensis* root extract, 1 part; hydrolyzed silk, 1 part; and dipotassium glycyrrhizinate, 10 parts.

The composition was prepared by mixing sodium hyaluronate, *Dendrobium candidum* stem extract, *Crocus sativus* extract, *Cereus grandiflorus* extract, *Centaurea cyanus* extract, *Scutellaria baicalensis* root extract, hydrolyzed silk and dipotassium glycyrrhizinate according to their mass ratios.

### Example 3

The raw material formula of a facial mask was: 84.11% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Dendrobium candidum* stem extract, 0.01% *Crocus sativus* extract, 0.01% *Cereus grandiflorus* extract, 0.01% *Centaurea cyanus* extract, 0.01% *Scutellaria baicalensis* root extract, 0.01% hydrolyzed silk, 0.1% dipotassium glycyrrhizinate, 0.2% chlorphenesin and 0.01% fragrance, and the above percentages refer to the mass percentage accounting for the total amount of the raw materials.

Preparation method of the facial mask: according to the above mass percentage, propylene glycol was homogenized in a homogenizer for 25 min, then hydroxyethyl cellulose, triethanolamine, sodium hyaluronate, dipotassium glycyrrhizinate, hydrolyzed silk and chlorphenesin were added, and the resulting mixture was homogenized for 25 min, and then water was added, and the resulting mixture was homogenized for 90 min, then PEG-40 hydrogenated castor oil, tocopheryl acetate and fragrance were added, and the resulting mixture was homogenized for 25 min, and finally, *Dendrobium candidum* stem extract, *Crocus sativus* extract, *Cereus grandiflorus* extract, *Centaurea cyanus* extract and *Scutellaria baicalensis* root extract were added, and the homogenized mixture was filtered through a 300 mesh filter screen.

### Effect Example 1

According to the SOD oxidase detection experiment, the composition prepared in Example 1 had a good antioxidant activity, which is more durable than that of the *Dendrobium candidum* stem extract alone.

According to the test for the moisture content of skin, the composition prepared in Example 1 had a good moisturizing property, which is more durable than that of the *Dendrobium candidum* stem extract alone.

### Effect Example 2

The indicators of the facial mask prepared with the composition in Example 3 are as follows:
(1) Sensory indicators
Color: transparent; traits: liquid; smell: faint scent; other: none.
(2) Hygienic chemical indicators

**Table 1 Hygienic chemical indicators**

| Test items | Indicators |
|---|---|
| Mercury | ≤ 1 mg/kg |
| Lead | ≤ 10 mg/kg |
| Arsenic | ≤ 2 mg/kg |
| Cadmium | ≤ 5 mg/kg |
| Dioxane | ≤ 30 mg/kg |

(3) Microbial indicators

**Table 2 Microbial indicators**

| Test items | Indicators |
|---|---|
| Colony forming unit | ≤ 1000 CFU/g |
| Total count of yeast and mould | ≤ 100 CFU/g |
| *Fecal coliform* | ND/g |
| *Pseudomonas aeruginosa* | ND/g |
| *Staphylococcus aureus* | ND/g |

(4) Test methods

**Table 3 Test methods**

| Test items | | Test methods |
|---|---|---|
| Hygienic chemical indicators | Mercury | Method for the determination of mercury - Method 2 - Hydride atomic spectrophotometry, "Hygienic Chemical Test Method" in "Hygienic Standard for Cosmetics" (2007 Edition). |
| | Lead | Method for the determination of lead - Method 1 - Flame atomic absorption spectrophotometry, "Hygienic Chemical Test Method" in "Hygienic |
| | | Standard for Cosmetics" (2007 Edition). |
| | Arsenic | Method for the determination of arsenic - Method 1 - Hydride atomic spectrophotometry, "Hygienic Chemical Test Method" in "Hygienic Standard for Cosmetics" (2007 Edition). |
| Microbial indicators | Colony forming unit | "Microbiological Testing Methods" in "Hygienic Standard for Cosmetics" (2007 Edition). |
| | Total count of yeast and mould | "Microbiological Testing Methods" in "Hygienic Standard for Cosmetics" (2007 Edition). |
| | *Fecal coliform* | "Microbiological Testing Methods" in "Hygienic Standard for Cosmetics" (2007 Edition). |
| | *Staphylococcus aureus* | "Microbiological Testing Methods" in "Hygienic Standard for Cosmetics" (2007 Edition). |
| | *Pseudomonas aeruginosa* | "Microbiological Testing Methods" in "Hygienic Standard for Cosmetics" (2007 Edition). |

(5) How to use
After cleansing, the facial mask was spread over the face and smoothed by hands.
(6) Safety assessment of risk substances

**Table 4**

| Serial number of raw materials | Standard Chinese name of raw materials | Is there a safety risk substance? | Remark |
|---|---|---|---|
| 1 | Water | × | No safety risk substance was detected. |
| 2 | Propylene glycol | × | No safety risk substance was detected. |
| 3 | Hydroxyethyl | × | No safety risk substance was detected. |
| | cellulose | | |
| 4 | Triethanolamine | √Dioxane, phenol | The residual amount of dioxane was in accordance with the requirement for the limit value of dioxane in cosmetics, which is tentatively set at not more than 30 mg/kg, proposed in "Announcement of the State Food and Drug Administration on the Limit Value of Dioxane in Cosmetics (No. 4) 2012" issued by SFDA on February 6, 2012. The residual amount of phenol was in accordance with the limited amount of phenol as a preservative: 0.1g/100g, specified in "Cosmetics Standard Schedule 3" of Notice No. 331 of the Ministry of Health and Welfare of Japan |
| 5 | PEG-40 hydrogenated castor oil | Dioxane | The residual amount of dioxane was in accordance with the requirement for the limit value of dioxane in cosmetics, which is tentatively set at not more than 30 mg/kg, proposed in "Announcement of the State Food and Drug Administration on the Limit Value of Dioxane in Cosmetics (No. 4) 2012" issued by SFDA on February 6, 2012. Thus, this product is safe. |
| 6 | Sodium hyaluronate | × | No safety risk substance was detected. |
| 7 | Tocopherol acetate | × | No safety risk substance was detected. |
| 8 | *Crocus sativus* extract | × | No safety risk substance was detected. |
| 9 | *Dendrobium candidum* stem extract | × | No safety risk substance was detected. |
| 10 | *Cereus grandiflorus* extract | × | No safety risk substance was detected. |
| 11 | *Centaurea cyanus* extract | × | No safety risk substance was detected. |
| 12 | *Scutellaria baicalensis* root extract | × | No safety risk substance was detected. |
| 13 | Hydrolyzed silk | × | No safety risk substance was detected. |
| 14 | Dipotassium glycyrrhizinate | × | No safety risk substance was detected. |
| 15 | Chlorphenesin | × | No safety risk substance was detected. |
| 16 | Fragrance | × | No safety risk substance was detected. |

(7) Instant use effect of the facial mask
40 volunteers aged between 30 and 40 years old were selected. After facial cleansing, they used the facial mask prepared in Example 3 of this application. The scores after use are as follows.

**Table 5**

| | |
|---|---|
| 5 points | The skin is moist, delicate, smooth, plump, shiny, soft and silky, and the facial contour is perfect and soft. |
| 4 points | The skin is moist and shiny. |
| 3 points | The skin is moist. |
| 2 points or less | No significant improvement on skin is found. |

**Table 6**

| | |
|---|---|
| 5 points | 38 |
| 4 points | 2 |
| 3 points | 0 |
| 2 points or less | 0 |

It can be seen from Tables 5 and 6 that after using the facial mask prepared in Example 3 of this application, the skin may be moist, delicate, smooth, plump, shiny, soft and silky, and the facial contour may be perfect and soft. The score rate of 5 points reached 95%.
(8) Long-term use effect of the facial mask
Experiment on long-term moisturizing effect on facial skin: Twenty women aged between 30 and 40 were selected, who separately used the facial mask product prepared in Example 3 and a certain brand of hydrating cosmetic purchased on the market (as Comparative Example 1). The facial mask was applied once a day for the first three days, and then once every two days, for four weeks. Before use, the moisture content of skin was measured with a skin tester under controlled conditions of a constant temperature of 25°C and a relative humidity of 40% as a reference. The moisture content of skin was measured after one week, two weeks, and four weeks of use, and three weeks after the suspension of use. The results are shown in the table below.

**Table 7**

| | One week | Two weeks | Four weeks | After three weeks of suspension |
|---|---|---|---|---|
| Example 3 | 44 | 47 | 49 | 45 |
| Comparative Example 1 | 31 | 32 | 32 | 11 |

As can be seen from Table 7, based on the values measured by the skin tester before the experiment, the moisture content of skin was significantly increased after a predetermined period of use. Compared with the certain brand of hydrating cosmetic, the facial mask prepared by this application had better moisturizing performance. Moreover, even after three weeks of suspension, the skin remained the comparable moisture content as it did after one week of use, indicating that the facial mask prepared in Example 3 has a long-lasting moisturizing property.

The facial mask prepared by the composition formulae of Example 1 and Example 2 had an effect comparable to that of Example 3.

## Claims

1. A composition containing *Dendrobium candidum* extract, comprising the following raw materials in parts by mass: 4-6 parts of sodium hyaluronate, 0.5-1.5 parts of *Dendrobium candidum* stem extract, 0.5-1.5 parts of *Crocus sativus* extract, 0.5-1.5 parts of *Cereus grandiflorus* extract, 0.5-1.5 parts of *Centaurea cyanus* extract and 0.5-1.5 parts of *Scutellaria baicalensis* root extract.

2. The composition of claim 1, wherein the content of the sodium hyaluronate is 5 parts;
and/or, the content of the *Dendrobium candidum* stem extract is 1 part;
and/or, the content of the *Crocus sativus* extract is 1 part;
and/or, the content of the *Cereus grandiflorus* extract is 1 part;
and/or, the content of the *Centaurea cyanus* extract is 1 part;
and/or, the content of the *Scutellaria baicalensis* root extract is 1 part.

3. The composition of claim 1 or 2, wherein the composition further comprises one or more of a solvent, a humectant, a thickener, a pH adjuster, a solubilizer, an antioxidant, a skin conditioner, a preservative, and a fragrance.

4. The composition of claim 3, wherein the composition further comprises a solvent, and the solvent is water;
the composition further comprises a humectant, and the humectant is propylene glycol; and the mass ratio of the humectant to the *Dendrobium candidum* stem extract is (1400 to 1600):1, and preferably 1500:1;
the composition further comprises a thickener, and the thickener is hydroxyethyl cellulose; and the mass ratio of the thickener to the *Dendrobium candidum* stem extract is (30 to 50):1, and preferably 40:1;
the composition further comprises a pH adjuster, and the pH adjuster is triethanolamine;
the composition further comprises a solubilizer, and the solubilizer is PEG-40 hydrogenated castor oil; and the mass ratio of the solubilizer to the *Dendrobium candidum* stem extract is (4 to 6):1, and preferably 5:1;
the composition further comprises an antioxidant, and the antioxidant is tocopheryl acetate; and the mass ratio of the antioxidant to the *Dendrobium candidum* stem extract is (0.5 to 1.5):1, and preferably 1:1;
the composition further comprises a skin conditioner, and the skin conditioner is dipotassium glycyrrhizinate and/or hydrolyzed silk; and the mass ratio of the skin conditioner to the *Dendrobium candidum* stem extract is (1 to 11):1;
the composition further comprises a preservative, and the preservative is chlorphenesin; and the mass ratio of the preservative to the *Dendrobium candidum* stem extract is (15 to 25):1, and preferably 20:1; and
the composition further comprises a fragrance, and the mass ratio of the fragrance to the *Dendrobium candidum* stem extract is (0.5 to 1.5): 1, and preferably 1:1.

5. The composition of claim 1, wherein the composition comprises the following raw materials in parts by weight: 5 parts of sodium hyaluronate, 1 part of *Dendrobium candidum* stem extract, 1 part of *Crocus sativus* extract, 1 part of *Cereus grandiflorus* extract, 1 part of *Centaurea cyanus* extract and 1 part of *Scutellaria baicalensis* root extract.

6. The composition of claim 1, wherein, the composition comprises: 84.11% water, 15% propylene glycol, 0.4% hydroxyethyl cellulose, 0.01% triethanolamine, 0.05% PEG-40 hydrogenated castor oil, 0.01% tocopheryl acetate, 0.05% sodium hyaluronate, 0.01% *Dendrobium candidum* stem extract, 0.01% *Crocus sativus* extract, 0.01% *Cereus grandiflorus* extract, 0.01% *Centaurea cyanus* extract, 0.01% *Scutellaria baicalensis* root extract, 0.01% hydrolyzed silk, 0.1% dipotassium glycyrrhizinate, 0.2% chlorphenesin and 0.01% fragrance, and the above percentages refer to the mass percentage accounting for the total amount of the raw materials.

7. A preparation method of the composition of any one of claims 1 to 6, wherein the raw material components of the composition are mixed.

8. The preparation method of claim 7, wherein the raw materials contain other component(s) in addition to the sodium hyaluronate, the *Dendrobium candidum* stem extract, the *Crocus sativus* extract, the *Cereus grandiflorus* extract, the *Centaurea cyanus* extract and the *Scutellaria baicalensis* root extract, and the other component(s) is/are firstly mixed and then mixed with the sodium hyaluronate, the *Dendrobium candidum* stem extract, the *Crocus sativus* extract, the *Cereus grandiflorus* extract, the *Centaurea cyanus* extract and the *Scutellaria baicalensis* root extract.

9. A method for manufacturing a cosmetic, comprising providing the composition of any one of claims 1 to 6.

10. The method of claim 9, wherein the cosmetic is a facial mask.

11. The method of claim 10, wherein the raw materials of the facial mask are: a solvent, a humectant, a thickener, a pH adjuster, a solubilizer, an antioxidant, sodium hyaluronate, *Dendrobium candidum* stem extract, *Crocus sativus* extract, *Cereus grandiflorus* extract, *Centaurea cyanus* extract, *Scutellaria baicalensis* root extract, a skin conditioner, a preservative, and a fragrance.

12. The method of claim 11, wherein the solvent is water;
the humectant is propylene glycol; and the mass ratio of the humectant to the *Dendrobium candidum* stem extract is (1400 to 1600):1, and preferably 1500:1;
the thickener is hydroxyethyl cellulose; and the mass ratio of the thickener to the *Dendrobium candidum* stem extract is (30 to 50):1, and preferably 40:1;
the pH adjuster is triethanolamine;
the solubilizer is PEG-40 hydrogenated castor oil; and the mass ratio of the solubilizer to the *Dendrobium candidum* stem extract is (4 to 6):1, and preferably 5:1;
the antioxidant is tocopheryl acetate; and the mass ratio of the antioxidant to the *Dendrobium candidum* stem extract is (0.5 to 1.5):1, and preferably 1:1;
the skin conditioner is dipotassium glycyrrhizinate and/or hydrolyzed silk; and the mass ratio of the skin conditioner to the *Dendrobium candidum* stem extract is (1 to 11):1;
the preservative is chlorphenesin; and the mass ratio of the preservative to the *Dendrobium candidum* stem extract is (15 to 25):1, and preferably 20:1; and
the mass ratio of the fragrance to the *Dendrobium candidum* stem extract is (0.5 to 1.5): 1, and preferably 1:1.

13. The method of claim 11 or 12, wherein the preparation method of the facial mask includes the following steps:
(1) mixing a group A and a group B, and homogenizing to obtain a mixture 1; the group A includes the humectant, and the group B includes the thickener, the pH adjuster, the sodium hyaluronate, the skin conditioner and the preservative;
(2) mixing the mixture 1 in the step (1) with the solvent, and homogenizing to obtain a mixture 2;
(3) mixing the mixture 2 in the step (2) with a group C, and homogenizing to obtain a mixture 3; and the group C includes the solubilizer, the antioxidant and the fragrance; and
(4) mixing the mixture 3 in the step (3) with a group D, and homogenizing; and the group D includes the *Dendrobium candidum* stem extract, the *Crocus sativus* extract, the *Cereus grandiflorus* extract, the *Centaurea cyanus* extract and the *Scutellaria baicalensis* root extract.

14. The method of claim 13, wherein in the step (1), the method for mixing the group A and the group B is separately adding the raw materials of the group B to the group A; and preferably, separately adding the raw materials of the group B to the group A after homogenization, and the time for homogenizing the group A is 20 min;
in the step (1), the time for homogenizing is 25 min;
in the step (1), the mixture 1 is filtered; and the mesh number of the filter screen for filtration is 300 mesh;
in the step (2), the time for homogenizing is 60 to 90 min;
in the step (3), the time for homogenizing is 25 min;
in the step (4), the *Dendrobium candidum* stem extract, the *Crocus sativus* extract, the *Cereus grandiflorus* extract, the *Centaurea cyanus* extract and the *Scutellaria baicalensis* root extract are separately added to the mixture 3; and
in the step (4), the time for homogenizing is 20 min.

## Patentansprüche

1. Zusammensetzung, die einen *Dendrobium candidum-*Extrakt enthält, umfassend die folgenden Rohmaterialien in Masseteilen:
4-6 Teile Natriumhyaluronat, 0,5-1,5 Teile *Dendrobium candidum*-Stammextrakt, 0,5-1,5 Teile *Crocus sativus*-Extrakt, 0,5-1,5 Teile *Cereus grandiflorus-*Extrakt, 0,5-1,5 Teile *Centaurea cyanus*-Extrakt und 0,5-1,5 Teile *Scutellaria baicalensis*-Wurzelextrakt.

2. Zusammensetzung nach Anspruch 1, wobei der Gehalt an Natriumhyaluronat 5 Teile beträgt;
und/oder der Gehalt an *Dendrobium candidum*-Stammextrakt 1 Teil beträgt;
und/oder der Gehalt an *Crocus sativus-*Extrakt 1 Teil beträgt;
und/oder der Gehalt an *Cereus grandiflorus-*Extrakt 1 Teil beträgt;
und/oder der Gehalt an *Centaurea cyanus-*Extrakt 1 Teil beträgt;
und/oder der Gehalt an *Scutellaria baicalensis*-Wurzelextrakt 1 Teil beträgt.

3. Zusammensetzung nach Anspruch 1 oder 2, wobei die Zusammensetzung ferner ein Lösungsmittel, ein Feuchthaltemittel, ein Verdickungsmittel, einen pH-Einsteller, einen Lösungsvermittler, ein Antioxidationsmittel, einen Hautkonditionierer, ein Konservierungsmittel und/oder einen Duftstoff umfasst.

4. Zusammensetzung nach Anspruch 3, wobei die Zusammensetzung ferner ein Lösungsmittel umfasst, und das Lösungsmittel Wasser ist;
wobei die Zusammensetzung ferner ein Feuchthaltemittel umfasst, und das Feuchthaltemittel Propylenglykol ist; und
das Massenverhältnis des Feuchthaltemittels zum *Dendrobium candidum*-Stammextrakt (1400 bis 1600): 1 und
vorzugsweise 1500:1 ist;
wobei die Zusammensetzung ferner ein Verdickungsmittel umfasst, und das Verdickungsmittel Hydroxyethylcellulose ist; und
wobei das Massenverhältnis des Verdickungsmittels zum *Dendrobium candidum-*Stammextrakt (30 bis 50): 1 und vorzugsweise 40:1 ist;
wobei die Zusammensetzung ferner einen pH-Einsteller umfasst, und der pH-Einsteller Triethanolamin ist;
wobei die Zusammensetzung ferner einen Lösungsvermittler umfasst, und der Lösungsvermittler PEG-40 hydriertes Rizinusöl ist; und
wobei das Massenverhältnis des Lösungsvermittlers zum *Dendrobium candidum-*Stammextrakt (4 bis 6): 1 und vorzugsweise 5:1 ist;
wobei die Zusammensetzung ferner ein Antioxidans umfasst, und das Antioxidans Tocopherylacetat ist; und
wobei das Massenverhältnis des Antioxidationsmittels zum *Dendrobium candidum-*Stammextrakt (0,5 bis 1,5):1 und vorzugsweise 1:1 ist;
wobei die Zusammensetzung ferner einen Hautkonditionierer umfasst, und der Hautkonditionierer Dikalium Glycyrrhizinat und/oder hydrolysierte Seide ist; und
wobei das Massenverhältnis des Hautkonditionierers zum *Dendrobium candidum-*Stammextrakt (1 bis 11): 1 ist;
wobei die Zusammensetzung ferner ein Konservierungsmittel umfasst, und das Konservierungsmittel Chlorphenesin ist; und
wobei das Massenverhältnis des Konservierungsmittels zum *Dendrobium candidum-*Stammextrakt (15 bis 25): 1 und vorzugsweise 20:1 ist; und
wobei die Zusammensetzung ferner einen Duftstoff umfasst und das Massenverhältnis des Duftstoffs zum *Dendrobium candidum*-Stammextrakt (0,5 bis 1,5): 1 und vorzugsweise 1:1 ist.

5. Zusammensetzung nach Anspruch 1, wobei die Zusammensetzung die folgenden Rohstoffe in Gewichtsteilen umfasst: 5 Teile Natriumhyaluronat, 1 Teil *Dendrobium candidum-*Stammextrakt, 1 Teil *Crocus sativus-*Extrakt, 1 Teil *Cereus grandiflorus*-Extrakt, 1 Teil *Centaurea cyanus-*Extrakt und 1 Teil *Scutellaria baicalensis*-Wurzelextrakt.

6. Zusammensetzung nach Anspruch 1, wobei die Zusammensetzung umfasst:
84,11 % Wasser, 15 % Propylenglykol, 0,4 % Hydroxyethylcellulose, 0,01 % Triethanolamin, 0,05 % hydriertes PEG-40-Rizinusöl, 0,01 % Tocopherylacetat, 0,05 % Natriumhyaluronat, 0,01 % *Dendrobium candidum*-Stammextrakt, 0,01 % *Crocus sativus-*Extrakt, 0,01 % *Cereus grandiflorus-*Extrakt, 0,01 % *Centaurea cyanus-*Extrakt, 0,01 % *Scutellaria baicalensis*-Wurzelextrakt, 0,01 % hydrolysierte Seide, 0,1 % Dikalium Glycyrrhizinat, 0,2 % Chlorphenesin und 0,01 % Duftstoff, wobei sich die obigen Prozentangaben auf den Massenanteil beziehen, der auf die Gesamtmenge der Rohstoffe entfällt.

7. Verfahren zum Herstellen der Zusammensetzung nach einem der Ansprüche 1 bis 6, wobei die Rohstoffkomponenten der Zusammensetzung vermischt werden.

8. Verfahren zum Herstellen nach Anspruch 7, wobei die Rohstoffe zusätzlich zu dem Natriumhyaluronat, dem *Dendrobium candidum*-Stammextrakt, dem *Crocus sativus-*Extrakt*,* dem *Cereus grandiflorus-*Extrakt*,* dem *Centaurea cyanus-*Extrakt und dem *Scutellaria baicalensis*-Wurzelextrakt andere Komponente(n) enthalten, und wobei die andere(n) Komponente(n) zuerst vermischt und dann mit dem Natriumhyaluronat, dem *Dendrobium candidum*-Stammextrakt, dem *Crocus sativus-*Extrakt*,* dem *Cereus grandiflorus*-Extrakt, dem *Centaurea cyanus-*Extrakt und dem *Scutellaria baicalensis*-Wurzelextrakt vermischt wird/werden.

9. Verfahren zum Herstellen eines Kosmetikums, umfassend das Bereitstellen der Zusammensetzung nach einem der Ansprüche 1 bis 6.

10. Verfahren nach Anspruch 9, wobei das Kosmetikum eine Gesichtsmaske ist.

11. Verfahren nach Anspruch 10, wobei die Rohstoffe der Gesichtsmaske Folgende sind:
ein Lösungsmittel, ein Feuchthaltemittel, ein Verdickungsmittel, ein pH-Einsteller, ein Lösungsvermittler, ein Antioxidationsmittel, Natriumhyaluronat, *Dendrobium candidum-*Stammextrakt, *Crocus sativus-*Extrakt*, Cereus grandiflorus-*Extrakt, *Centaurea cyanus-*Extrakt, *Scutellaria baicalensis*-Wurzelextrakt, ein Hautkonditionierer, ein Konservierungsmittel und ein Duftstoff.

12. Verfahren nach Anspruch 11, wobei das Lösungsmittel Wasser ist;
wobei das Feuchthaltemittel Propylenglykol ist; und
das Massenverhältnis des Feuchthaltemittels zum *Dendrobium candidum*-Stammextrakt (1400 bis 1600): 1 und vorzugsweise 1500:1 ist;
wobei das Verdickungsmittel Hydroxyethylcellulose ist; und
wobei das Massenverhältnis des Verdickungsmittels zum *Dendrobium candidum-*Stammextrakt (30 bis 50): 1 und vorzugsweise 40:1 ist;
wobei der pH-Einsteller Triethanolamin ist;
wobei der Lösungsvermittler PEG-40 hydriertes Rizinusöl ist; und
wobei das Massenverhältnis des Lösungsvermittlers zum *Dendrobium candidum-*Stammextrakt (4 bis 6):1, vorzugsweise 5:1 ist;
wobei das Antioxidationsmittel Tocopherylacetat ist; und
wobei das Massenverhältnis des Antioxidationsmittels zum *Dendrobium candidum-*Stammextrakt (0,5 bis 1,5): 1, vorzugsweise 1:1 ist;
wobei der Hautkonditionierer Dikalium Glycyrrhizinat und/oder hydrolysierte Seide ist; und wobei das Massenverhältnis des Hautkonditionierers zum *Dendrobium candidum-*Stammextrakt (1 bis 11): 1 ist;
wobei das Konservierungsmittel Chlorphenesin ist; und
wobei das Massenverhältnis des Konservierungsmittels zum *Dendrobium candidum-*Stammextrakt (15 bis 25): 1 und vorzugsweise 20:1 ist;
wobei das Massenverhältnis des Duftstoffs zum *Dendrobium candidum*-Stammextrakt (0,5 bis 1,5): 1 und vorzugsweise 1:1 ist.

13. Verfahren nach Anspruch 11 oder 12, wobei das Verfahren zum Herstellen der Gesichtsmaske die folgenden Schritte beinhaltet:
(1) Mischen einer Gruppe A und einer Gruppe B, und Homogenisieren, um eine Mischung 1 zu erhalten;
wobei die Gruppe A das Feuchthaltemittel und die Gruppe B das Verdickungsmittel, den pH-Einsteller, das Natriumhyaluronat, den Hautkonditionierer und das Konservierungsmittel beinhaltet;
(2) Vermischen der Mischung 1 in Schritt (1) mit dem Lösungsmittel und Homogenisieren, um eine Mischung 2 zu erhalten;
(3) Vermischen der Mischung 2 in Schritt (2) mit einer Gruppe C und Homogenisieren, um eine Mischung 3 zu erhalten; und
wobei die Gruppe C den Lösungsvermittler, das Antioxidationsmittel und den Duftstoff beinhaltet; und
(4) Vermischen der Mischung 3 im Schritt (3) mit einer Gruppe D und Homogenisieren; und
wobei die Gruppe D den *Dendrobium candidum*-Stammextrakt, den *Crocus sativus-*Extrakt, den *Cereus grandiflorus*-Extrakt, den *Centaurea cyanus-*Extrakt und den *Scutellaria baicalensis*-Wurzelextrakt beinhaltet.

14. Verfahren nach Anspruch 13, wobei im Schritt (1) das Verfahren zum Vermischen der Gruppe A und der Gruppe B darin besteht, dass die Rohstoffe der Gruppe B der Gruppe A getrennt zugegeben werden; und
vorzugsweise die Rohstoffe der Gruppe B nach dem Homogenisieren der Gruppe A zugegeben werden, wobei die Zeit zum Homogenisieren der Gruppe A 20 min beträgt;
wobei die Zeit zum Homogenisieren in Schritt (1) 25 min beträgt;
wobei das Gemisch 1 in Schritt (1) gefiltert wird; und
wobei die Mesh-Zahl des Filtersiebs für die Filtration 300 Mesh beträgt;
wobei die Zeit zum Homogenisieren im Schritt (2) 60 bis 90 min beträgt;
wobei die Zeit zum Homogenisieren in Schritt (3) 25 min beträgt;
wobei der *Dendrobium candidum*-Stammextrakt, der *Crocus sativus-*Extrakt*,* der *Cereus grandiflorus*-Extrakt, der *Centaurea cyanus-*Extrakt und der *Scutellaria baicalensis-*Wurzelextrakt in Schritt (4) der Mischung 3 getrennt zugegeben werden; und
wobei die Zeit zum Homogenisieren in Schritt (4) 20 min beträgt.

## Revendications

1. Composition contenant un extrait de *Dendrobium candidum,* comprenant les matières premières suivantes en parties en masse :
4 à 6 parties d'hyaluronate de sodium, 0,5 à 1,5 parties d'extrait de tige de *Dendrobium candidum,* 0,5 à 1,5 parties d'extrait de *Crocus sativus,* 0,5 à 1,5 parties d'extrait de *Cereus grandiflorus,* 0,5 à 1,5 parties d'extrait de *Centaurea cyanus* et 0,5 à 1,5 parties d'extrait de racine de *Scutellaria baicalensis.*

2. Composition selon la revendication 1, dans laquelle la teneur en hyaluronate de sodium est de 5 parties ;
et/ou, le contenu de l'extrait de tige de *Dendrobium candidum* est de 1 partie ;
et/ou, la teneur en extrait de *Crocus sativus* est de 1 partie ;
et/ou, la teneur en extrait de *Cereus grandiflorus* est de 1 partie ;
et/ou, la teneur en extrait de *Centaurea cyanus* est de 1 partie ;
et/ou, la teneur en extrait de racine de *Scutellaria baicalensis* est de 1 partie.

3. Composition selon la revendication 1 ou 2, la composition comprenant en outre un ou plusieurs parmi un solvant, un humectant, un épaississant, un ajusteur de pH, un solubilisant, un antioxydant, un revitalisant pour la peau, un conservateur et un parfum.

4. Composition selon la revendication 3, la composition comprenant en outre un solvant, et le solvant est de l'eau ;
la composition comprend en outre un humectant, et l'humectant est du propylène glycol ; et
le rapport massique entre l'humectant et l'extrait de tige de *Dendrobium candidum* est de (1 400 à 1 600):1, et
de préférence de 1 500:1 ;
la composition comprend en outre un épaississant et l'épaississant est l'hydroxyéthylcellulose ; et
le rapport massique entre l'épaississant et l'extrait de tige de *Dendrobium candidum* est de (30 à 50):1, et de préférence 40:1 ;
la composition comprend en outre un ajusteur de pH, et l'ajusteur de pH est la triéthanolamine ;
la composition comprend en outre un solubilisant, et le solubilisant est l'huile de ricin hydrogénée PEG-40 ; et
le rapport massique entre solubilisant et l'extrait de tige de *Dendrobium candidum* est de (4 à 6):1, et de préférence de 5:1 ;
la composition comprend en outre un antioxydant, et l'antioxydant est l'acétate de tocophéryle ; et
le rapport massique entre l'antioxydant et l'extrait de tige de *Dendrobium candidum* est de (0,5 à 1,5):1, et de préférence de 1:1 ;
la composition comprend en outre un revitalisant pour la peau, et le revitalisant pour la peau est du glycyrrhizinate de dipotassium et/ou de la soie hydrolysée ; et
le rapport massique entre le revitalisant pour la peau et l'extrait de tige de *Dendrobium candidum* est de (1 à 11):1 ;
la composition comprend en outre un conservateur, et le conservateur est la chlorphénésine ; et
le rapport massique entre le conservateur et l'extrait de tige de *Dendrobium candidum* est de (15 à 25):1, et de préférence de 20:1 ; et
la composition comprend en outre un parfum, et le rapport massique entre le parfum et l'extrait de tige de *Dendrobium candidum* est de (0,5 à 1,5):1, et de préférence de 1:1.

5. Composition selon la revendication 1, la composition comprenant les matières premières suivantes en parties en poids : 5 parties d'hyaluronate de sodium, 1 partie d'extrait de tige de *Dendrobium candidum,* 1 partie d'extrait de *Crocus sativus,* 1 partie d'extrait de *Cereus grandiflorus,* 1 partie d'extrait de *Centaurea cyanus* et 1 partie d'extrait de racine de *Scutellaria baicalensis.*

6. Composition selon la revendication 1, la composition comprenant :
84,11 % d'eau, 15 % de propylène glycol, 0,4 % d'hydroxyéthylcellulose, 0,01 % de triéthanolamine, 0,05 % d'huile de ricin hydrogénée PEG-40, 0,01 % d'acétate de tocophéryle, 0,05 % d'hyaluronate de sodium, 0,01 % d'extrait de tige de *Dendrobium candidum,* 0,01 % d'extrait de *Crocus sativus,* 0,01 % d'extrait de *Cereus grandiflorus,* 0,01 % d'extrait de *Centaurea cyanus,* 0,01 % d'extrait de racine de *Scutellaria baicalensis,* 0,01 % de soie hydrolysée, 0,1 % de glycyrrhizinate dipotassique, 0,2 % de chlorphénésine et 0,01 % de parfum, et les pourcentages ci-dessus se réfèrent au pourcentage massique représentant la quantité totale de matières premières.

7. Procédé de préparation de la composition selon l'une quelconque des revendications 1 à 6, dans lequel les composants des matières premières de la composition sont mélangés.

8. Procédé de préparation selon la revendication 7, dans lequel les matières premières contiennent d'autres composants en plus de l'hyaluronate de sodium, de l'extrait de tige de *Dendrobium candidum,* de l'extrait de *Crocus sativus,* de l'extrait de *Cereus grandiflorus,* de l'extrait de *Centaurea cyanus* et de l'extrait de racine de *Scutellaria baicalensis.,* et l'autre/les autres composant(s) est/sont d'abord mélangé(s), puis mélangé(s) avec l'hyaluronate de sodium, l'extrait de tige de *Dendrobium candidum,* l'extrait de *Crocus sativus,* l'extrait de *Cereus grandiflorus,* l'extrait de *Centaurea cyanus* et l'extrait de racine de *Scutellaria baicalensis.*

9. Procédé de production d'un cosmétique, comprenant la fourniture de la composition selon l'une quelconque des revendications 1 à 6.

10. Procédé selon la revendication 9, dans lequel le cosmétique est un masque facial.

11. Procédé selon la revendication 10, dans lequel les matières premières du masque facial sont :
un solvant, un humectant, un épaississant, un ajusteur de pH, un solubilisant, un antioxydant, un hyaluronate de sodium, un extrait de tige de *Dendrobium candidum,* un extrait de *Crocus sativus,* un extrait de *Cereus grandiflorus,* un extrait de *Centaurea cyanus,* un extrait de racine de *Scutellaria baicalensis,* un revitalisant pour la peau, un conservateur et un parfum.

12. Procédé selon la revendication 11, dans lequel le solvant est de l'eau ;
l'humectant est le propylène glycol ; et
le rapport massique entre l'humectant et l'extrait de tige de *Dendrobium candidum* est de (1 400 à 1 600):1, et
de préférence de 1 500:1 ;
l'épaississant est l'hydroxyéthylcellulose ; et
le rapport massique entre l'épaississant et l'extrait de tige de *Dendrobium candidum* est de (30 à 50):1, et de préférence 40:1 ;
l'ajusteur de pH est la triéthanolamine ;
le solubilisant est de l'huile de ricin hydrogénée PEG-40 ; et
le rapport massique entre le solubilisant et l'extrait de tige de *Dendrobium candidum* est de (4 à 6):1, et de préférence de 5:1 ;
l'antioxydant est l'acétate de tocophéryle ; et
le rapport massique entre l'antioxydant et l'extrait de tige de *Dendrobium candidum* est de (0,5 à 1,5):1, et de préférence de 1:1 ;
le revitalisant pour la peau est du glycyrrhizinate de dipotassium et/ou de la soie hydrolysée ; et
le rapport massique entre le revitalisant pour la peau et l'extrait de tige de *Dendrobium candidum* est de (1 à 11):1 ;
le conservateur est la chlorphénésine ; et
le rapport massique entre le conservateur et l'extrait de tige de *Dendrobium candidum* est de (15 à 25):1,
et de préférence de 20:1 ; et
le rapport massique entre le parfum et l'extrait de tige de *Dendrohium candidum* est de (0,5 à 1,5):1, et de préférence de 1:1.

13. Procédé selon la revendication 11 ou 12, le procédé de préparation du masque facial comportant les étapes suivantes :
(1) le mélange d'un groupe A et d'un groupe B, et l'homogénéisation de ceux-ci pour obtenir un mélange 1 ;
le groupe A comporte l'humectant, et le groupe B comporte l'épaississant, l'ajusteur de pH, l'hyaluronate de sodium, le revitalisant pour la peau et le conservateur ;
(2) le mélange du mélange 1 à l'étape (1) avec le solvant, et l'homogénéisation de ceux-ci pour obtenir un mélange 2 ;
(3) le mélange du mélange 2 à l'étape (2) avec un groupe C, et l'homogénéisation de ceux-ci pour obtenir un mélange 3 ; et
le groupe C comporte le solubilisant, l'antioxydant et le parfum ; et
(4) le mélange du mélange 3 à l'étape (3) avec un groupe D, et l'homogénéisation de ceux-ci ; et
le groupe D comporte l'extrait de tige de *Dendrobium candidum,* l'extrait de *Crocus sativus,* l'extrait de *Cereus grandiflorus,* l'extrait de *Centaurea cyanus* et l'extrait de racine de *Scutellaria baicalensis.*

14. Procédé selon la revendication 13, dans lequel, à l'étape (1), le procédé de mélange du groupe A et du groupe B consiste à ajouter séparément les matières premières du groupe B au groupe A ; et
de préférence, à ajouter séparément les matières premières du groupe B au groupe A après homogénéisation, et le temps d'homogénéisation du groupe A est de 20 min ;
à l'étape (1), le temps d'homogénéisation est de 25 min ;
à l'étape (1), le mélange 1 est filtré ; et
le nombre de mailles du tamis filtrant pour la filtration est de 300 mailles ;
à l'étape (2), le temps d'homogénéisation est de 60 à 90 min ;
à l'étape (3), le temps d'homogénéisation est de 25 min ;
à l'étape (4), l'extrait de tige de *Dendrobium candidimi,* l'extrait de *Crocus sativus,* l'extrait de *Cereus grandiflorus,* l'extrait de *Centaurea cyanus* et l'extrait de racine de *Scutellaria baicalensis* sont ajoutés séparément au mélange 3 ; et
à l'étape (4), le temps d'homogénéisation est de 20 min.
